# EUROPEAN PATENT APPLICATION

(11) **EP 3 042 579 A1**
(43) Date of publication of application: **13.07.2016**
(21) Application number: 15150571.6
(22) Date of filing: 09.01.2015
(51) Int. Cl.: A24F 47/00

(54) **Electronic smoking device**

(71) Applicant: Fontem Holdings 1 B.V., 1083 HN Amsterdam (NL)
(72) Inventor: Borkovec, Vaclav, 22761 Hamburg (DE); Gonzales, Diego, 1083 Amsterdam (NL)
(74) Representative: Gulde & Partner

(57) **Abstract**

An electronic smoking device (10) has a mouthpiece portion (14) and a main body (12). The mouthpiece portion (14) has an air inhalation port (36) and houses an atomizer (26). The main body (12) has at least one air inlet (38) and houses an electronic element (22, 24). The electronic element (22, 24) has at least one of control electronics and an airflow sensor. A first hydraulic diameter of the at least one air inlet (38) is larger than a second hydraulic diameter of a bottleneck (41, 141) in a conductive part arranged between the electronic element (22, 24) and the atomizer (26).

## Description

### FIELD OF INVENTION

The present invention relates generally to electronic smoking devices and in particular electronic cigarettes.

### BACKGROUND OF THE INVENTION

An electronic smoking device, such as an electronic cigarette (e-cigarette), usually has a housing accommodating an electric power source (e.g. a single use battery or a rechargeable battery), and an electrically operable atomizer. The atomizer vaporizes or atomizes liquid supplied from a reservoir and provides vaporized or atomized liquid as an aerosol. Control electronics control the activation of the atomizer. In many electronic cigarettes, an airflow sensor is provided within the electronic smoking device which detects a user puffing on the device (e.g., by sensing an underpressure or an air flow pattern through the device). The airflow sensor indicates or signals the puff to the control electronics. Alternatively, a button may be used to switch on the electronic smoking device to generate a puff of vapour. When a puff is detected, the control electronics supplies electrical power to the atomizer thereby creating vaporized liquid as an aerosol.

In some electronic smoking devices the vaporized or atomized liquid is provided as aerosol in a central passage from an inlet to an air inhalation port where the user puffs on the device thereby generating the flow of gas.

### SUMMARY OF THE INVENTION

Aspects of the invention concern embodiments as set forth in the claims.

In one aspect an electronic smoking device has a mouthpiece portion and a main body. The mouthpiece portion comprises an air inhalation port and houses an atomizer. The main body comprises at least one air inlet and houses an electronic element. The electronic element comprises at least one of control electronics and an airflow sensor. A first hydraulic diameter of the at least one air inlet is larger than a second hydraulic diameter of a bottleneck in a conductive part arranged between the electronic element and the atomizer.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
- Figure 1: is a schematic cross-sectional illustration of an exemplary e-cigarette;
- Figure 2: shows a first embodiment of the invention,
- Figure 3: shows a second embodiment of the invention,
- Figure 4: shows an optional realization of a plate according to the first embodiment,
- Figure 5: shows an optional realization of the plate according to the second embodiment,
- Figure 6: shows an enlarged detail of figure 4 and
- Figure 7: shows an enlarged detail of figure 5.

### DESCRIPTION OF THE PREFERRED EMBODIMENTS

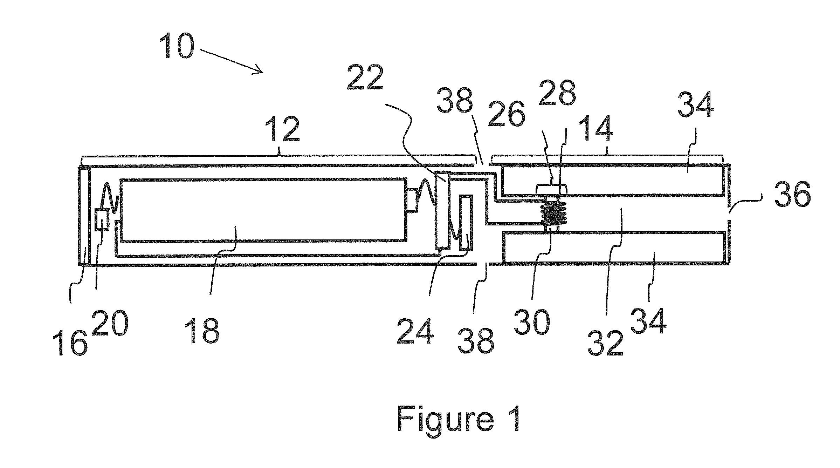

Prior to describing an embodiment of the present invention, an example of a basic structure of an e-cigarette will first be described with reference to Figure 1 which is a schematic cross-sectional illustration of an exemplary e-cigarette.

As is shown in Figure 1, an e-cigarette 10 ordinarily comprises a cylindrical housing having a main body 12 and a mouthpiece portion 14. Together the main body 12 and the mouthpiece portion 14 form a cylindrical tube which is approximately the same size and shape as a conventional cigarette. The main body 12 and mouthpiece 14 are typically made of steel or hardwearing plastic and act to provide a housing to contain the operative elements of the e-cigarette 10. The main body 12 and a mouthpiece portion 14 may be configured to fit together by means of a friction push fit. Alternatively in some embodiments a screw fit may be provided enabling the main body 12 and mouthpiece portion 14 to be attached to one another.

The mouthpiece portion may be removable from the main body, or the mouthpiece portion may be part of, or integral with, the main body. In embodiments where the mouthpiece portion is a component separate from the main body, the mouthpiece portion may be attached to the main body via a friction push fit, a snap fit, a bayonet attachment or screw threads. In a further aspect, the main body and the mouthpiece portion, in whichever form provided, together comprise an electronic smoking device which is approximately the same size and shape as a conventional tobacco cigarette, typically about 100 mm with a 7.5 mm diameter, although lengths may range from 70 to 150 or 180 mm, and diameters from 5 to 20 mm.

An end cap 16 may be provided at the end of the main body 12 remote from the mouthpiece portion 14 enclosing that end of the main body 12. The end cap 16 is typically made from translucent plastic.

A battery 18 is provided within the central cavity enclosed by the main body 12. In a typical design, also contained within the central cavity defined by the main body 12 are a light emitting diode (LED) 20, control electronics 22 and an airflow sensor 24. The battery 18 is electrically connected to the LED 20 and the control electronics 22 and the airflow sensor 24 is connected to the control electronics 22. In this example the LED 20 is provided at one end of the main body 12, adjacent to the end cap 16 and the control electronics 22 and airflow sensor 24 are provided in the central cavity at the other end of the battery 18 adjacent the mouth piece portion 14.

The airflow sensor 24 acts as a puff detector, detecting a user puffing or sucking on the mouthpiece portion 14 of the e-cigarette 10. The airflow sensor 24 can be any suitable sensor for detecting changes in airflow or air pressure such a microphone switch including a deformable membrane which is caused to move by variations in air pressure.

The control electronics 22 are also connected to an atomizer 26 which in this illustrative example comprises a heating coil 28 which is wrapped around a wick 30 which extends across a central passage 32 provided in the mouthpiece portion 14 of the e-cigarette 10. The dimensions of the central passage 32, the wick 30 and the heating coil 28 are such that the wick 30 and heating coil 28 do not completely block the central passage 32 but rather an air gap is provided either side of the heating coil 28 enabling air to flow past the heating coil 28 and wick 30.

The central passage 32 is surrounded by a cylindrical liquid store 34 with the ends of the wick 30 abutting or extending into the liquid store 34. The wick 30 comprises a porous material such as a bundle of fibreglass fibres such that liquid present in the liquid store 34 is drawn by capillary action from the ends of the wick 30 towards the central portion of the wick 30 encircled by the heating coil 28.

In some embodiments the liquid store 34 will comprise wadding soaked in liquid which encircles the central passage 32 with the ends of the wick 30 abutting the wadding. In other embodiments the liquid store 34 may comprise a toroidal cavity arranged to be filled with liquid to be vaporized with the toroidal cavity enclosed by walls and with the ends of the wick 30 extending into the toroidal cavity.

An air inhalation port 36 is provided at the end of the mouthpiece portion 14 remote from main body 12 of the e-cigarette 10 and a pair of air inlets 38 are provided in the housing at the intersection between the main body 12 and the mouthpiece portion 14 adjacent the airflow sensor 24 with the central passage 32 within the mouthpiece portion 14 of the e-cigarette 10 extending from adjacent the air inlets 38 past the wick 30 and heating coil 28 to the air inhalation port 36.

In use, a user sucks on the mouthpiece portion 14 of the e-cigarette 10. This causes air to be drawn into the e-cigarette 10 via the air inlets 38 and to be drawn up via the central passage 32 towards the air inhalation port 36. The change in air pressure which arises is detected by the airflow sensor 24 which generates an electrical signal that is passed to the control electronics 22. In response to the signal, the control electronics 22 then proceed to activate the heating coil 28 which causes liquid present in the wick 30 to be vaporized creating an aerosol (which may comprise gaseous and liquid components) within the central passage 32. As the user continues to suck on the mouthpiece portion 14 of the e-cigarette 10, this aerosol is drawn up the central passage 32 and inhaled by the user sucking on the e-cigarette 10. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid store 34 by capillary action and thus is available to be converted into an aerosol through subsequent activation of the heating coil 28.

In some e-cigarettes, the battery 18 is rechargeable and a means is provided to replenish the liquid supply. In the cases where the liquid store 34 is a toroidal cavity, this may be achieved by providing a refill port and refilling the cavity with liquid via the refill port. In other embodiments the mouthpiece portion 14 of the e-cigarette 10 is detachable from the main body 12 and a new mouthpiece portion 14 can be fitted with a new liquid store 34 thereby replenishing the supply of liquid. In some cases, replacing the liquid store 34 may involve replacement of the heating coil 28 and the wick 30 along with the replacement of the liquid store 34.

In some cases the new liquid store 34 may be in the form of a cartridge. In some such embodiments the cartridge may be provided with a central passage 32 through which a user inhales aerosol generated by the e-cigarette. In other embodiments, rather than inhaling aerosol via a central passage 32, the cartridge may be such to block the central portion of the e-cigarette 10 and generated aerosol may be directed around the exterior of the cartridge 32 to an air inhalation port 36 for inhalation.

It will also be appreciated that although the above description is illustrative of the structure and function of a typical e-cigarette 10, variations also exist. Thus for example in some e-cigarettes the LED 20 is omitted. In some e-cigarettes, the airflow sensor 24 may be placed adjacent the end cap 16 of the e-cigarette rather than in the middle of the e-cigarette as illustrated. Similarly, in some e-cigarettes, the air inlets 36 may be placed at the distal end of the main body 16 of the e-cigarette 10 remote from the mouthpiece portion 14.. In designs not using a separate mouthpiece portion, the inhalation port 36 may be provided at the back end of the main body 12. The location and number of inlets 38 may vary. A single inlet may be used. The inlet or inlets 38 may be on or in the main body, or the mouthpiece portion, or both.

In some e-cigarettes the airflow sensor 24 is omitted and instead a button is provided which enables a user to activate the e-cigarette manually rather than in response to the detection of a change in air flow or air pressure. Also in some e-cigarettes the constitution of the atomizer may be changed. Thus for example rather than being constituted by a wick 28 surrounded by a heating coil 26 other configurations may be used such as providing a heating coil in the interior of a porous body and generating an aerosol by heating air and passing the air through the porous body. The atomizer may alternatively be provided with other forms of heating elements, such as ceramic heaters, or fiber or mesh material heaters. Non resistance heating elements such as sonic and jet spray may also be used in the atomizer in place of the heating coil. Further in some embodiments rather than generating an aerosol through heating liquid within a wick 28 an aerosol might be generated using a piezoelectric atomizer to create an aerosol for inhalation either in combination or in the absence of a heater.

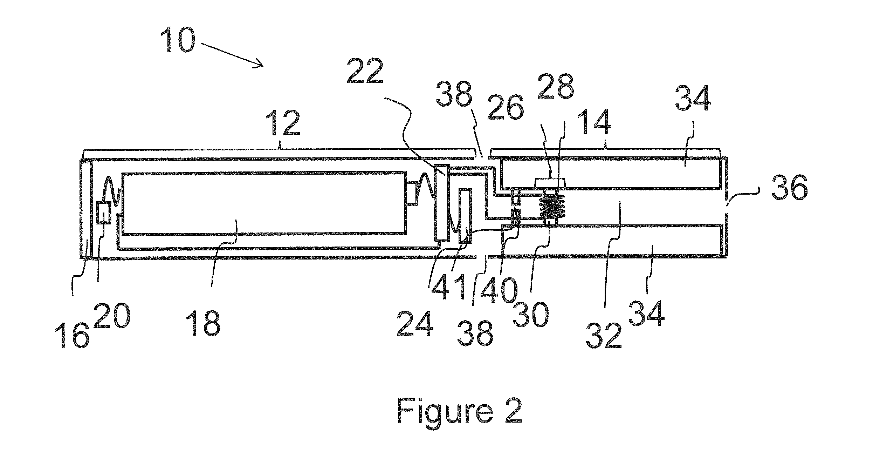

Figure 2 shows a first embodiment of the invention. The second embodiment comprises some same or like elements as described within the context of figure 1. A repetition of the description of the same or like elements is omitted here.

A plate 40 is arranged in the mouthpiece portion 32 in the central passage 32. The plate 40 comprises a hole 41. The through hole 41 forms a bottleneck 41. The bottleneck 41 has a second hydraulic diameter smaller than a first hydraulic diameter of the air inlet 38.

Due to the diameter difference, pressure drop in use between the air inlets and the air inhalation port occurs mainly at the bottleneck. This has the effect that in use a back flow is reduced or restricted. The back flow is a flow of at least one of the aerosol and a condensate of the aerosol into the main body. The back flow is reduced with respect to a further back flow occurring in an electronic smoking device where the second hydraulic diameter is equal to or less than the first hydraulic diameter. Since the back flow is reduced or restricted, less or no condensate forms and/or flows into the main body. A risk is reduced of one or more of the electric energy providing element and the airflow sensor being damaged by the condensate.

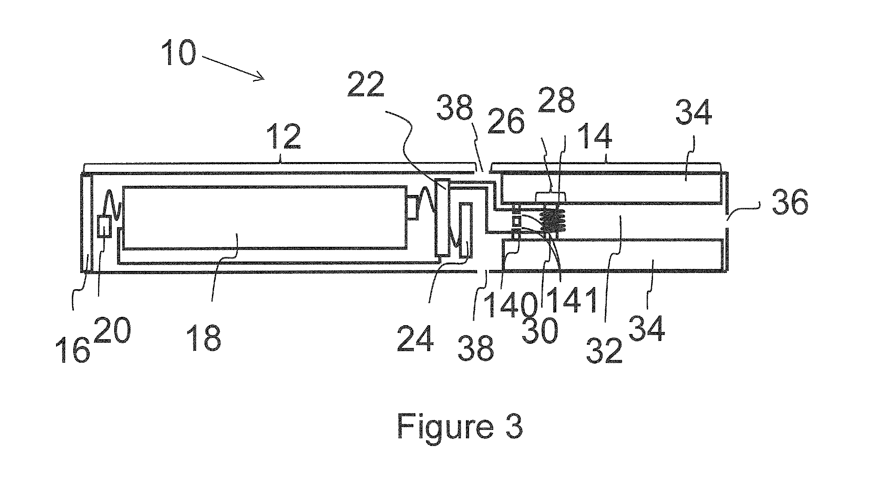

Figure 3 shows a second embodiment of the invention. The second embodiment comprises some same or like elements as the first embodiment or as described within the context of figure 1. A repetition of the description of the same or like elements is omitted here.

The second embodiment differs from the first embodiment in that the bottleneck 141 comprises several through holes 41. Figure 3 depicts two through holes 41. Optionally, three, four or more through holes are present. Each of the through holes has a through hydraulic diameter. A sum of the through holes' hydraulic diameters equals the second hydraulic diameter.

The second embodiment provides the same effect of reduced or restricted back flow of at least one of the aerosol and the condensate.

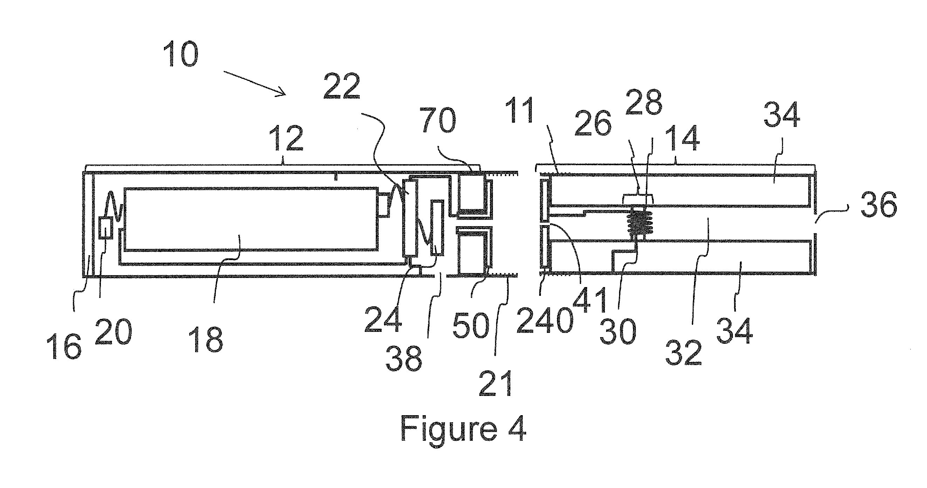

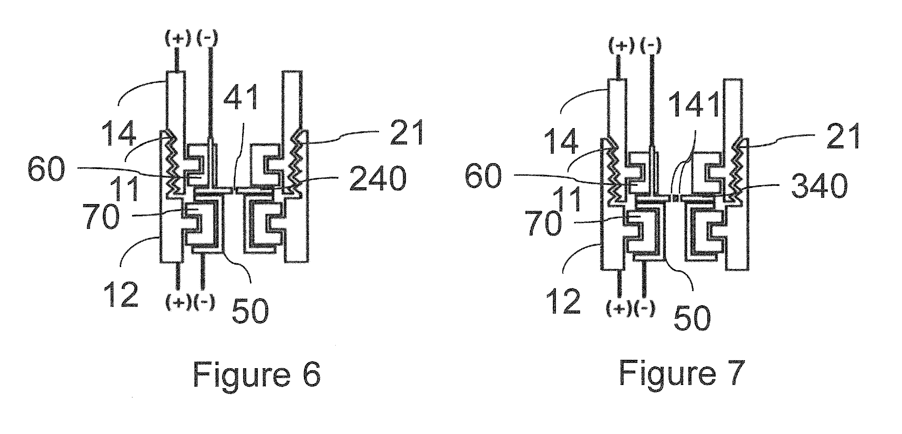

Figure 4 shows an optional realization of the plate according to the first embodiment of the invention. Figure 6 shows an enlarged detail of figure 4.

In the embodiment shown in figure 4, the mouthpiece portion 14 and the main body 12 are conductive. The mouthpiece portion 14 is electrically connected to the atomizer 26 and the main body 12 electrically is connected to the control electronics 22. The atomizer 26 is further electrically connected to a conductive part arranged within the mouthpiece portion 14. The control electronics 22 is further electrically connected to a further conductive part 50 arranged within the main body 12. In figure 4, the mouthpiece portion 14 and the conductive element are electrically isolated from each other by means of the liquid store 34 arranged between the two.

Or, an isolation element 60 may be arranged between the mouthpiece portion 14 and the conductive element as shown in Figure 6. A further isolation element 70 is arranged between the main body 12 and the further conductive element 50.

An electrical connection of the control electronics 22 with the atomizer 26 results from fitting together the mouthpiece portion 14 and the main body 12.

In the embodiment shown in figure 4 the conductive part is at least partly formed as the plate 240 with the through hole. The plate is then easily accessible for cleaning the through holes.

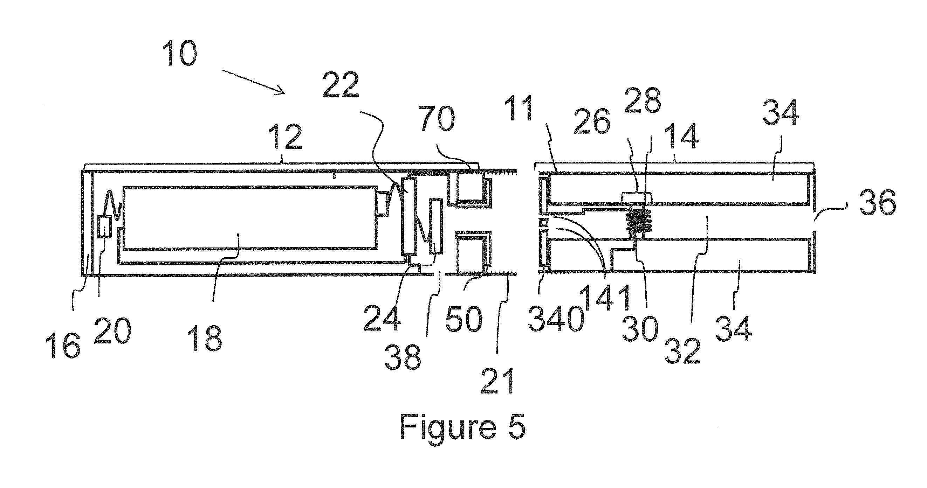

Figure 5 shows an optional realization of the plate according to the second embodiment providing the same advantage of easy cleaning. Figure 7 shows an enlarged detail of figure 5. In the embodiment depicted in Figure 5 and 7, the plate comprises some same or like elements as the embodiment of the plate depicted in Figure 4 and 6.

In the embodiment shown in figure 5 the conductive part is at least partly formed as the plate 340 with the through holes.

Figures 5 and 7 depict two through holes. Optionally, three, four or more through holes are present. Each of the through holes has a hydraulic diameter. A sum of the holes' hydraulic diameters equals the second hydraulic diameter.

In the embodiments shown in figures 4 - 7 at least one of the main body 12 and the further conductive part 50 may be electrically connected to the electric energy providing element 18 via the control electronics. Then, the control electronics may be configured to control electrical connection between the electric energy providing element 18 and the at least one of the main body 12 and the further conductive part 50. The control electronics is optional.

In a first variant of the various embodiments, the diameter of each bore hole is not greater than 0.05 mm. In a second variant of the various embodiments diameter of each bore hole is not greater than 0.04 mm. In a third variant of the various embodiments, the diameter of each bore hole is not greater than 0.03 mm. In a fourth variant of the various embodiments, the diameter of each bore hole is not greater than 0.02 mm. In a fifth variant of the various embodiments, the diameter of each bore hole is not greater than 0.01 mm. In each of these variants, the bore holes are round. Non-round bore holes may also be used, with their hydraulic diameters calculated using known techniques.

The smaller the diameter, the better back flow is prevented.

The airflow sensor 24 may be a pressure sensitive switch which is activated by a pressure difference between two ends of the airflow sensor 24.

For fitting together, the mouthpiece portion 14 may comprises an external screw thread 11 and the main body 12 may comprise a corresponding internal screw thread 21.

While this invention has been described in connection with what is presently considered to be practical exemplary embodiments, it is to be understood that the present invention is not limited to the disclosed embodiments, but, on the contrary, is intended to cover various modifications and equivalent arrangements included within the scope of the appended claims.

## Claims

1. An electronic smoking device (10) comprising
a mouthpiece portion (14) having an air inhalation port (36) and housing an atomizer (26),
a main body (12) having at least one air inlet (38) and housing an electronic element (22, 24), the electronic element comprising at least one of control electronics (22) and an airflow sensor (24), wherein a first hydraulic diameter of the at least one air inlet (38) is larger than a second hydraulic diameter of a bottleneck (41, 141) in a conductive part arranged between the electronic element (22, 24) and the atomizer (26).

2. Electronic smoking device of claim 1 wherein the second hydraulic diameter is not greater than 0.05 mm.

3. Electronic smoking device of claim 2 wherein the second hydraulic diameter is not greater than 0.04 mm.

4. Electronic smoking device of claim 3 wherein the second hydraulic diameter is not greater than 0.03 mm.

5. Electronic smoking device of claim 4 wherein the second hydraulic diameter is not greater than 0.02 mm.

6. Electronic smoking device of claim 5 wherein the second hydraulic diameter is not greater than 0.01 mm.

7. Electronic smoking device of one of the preceding claims wherein the bottleneck (41, 141) is formed by at least one through hole through a conductive plate (40, 140, 240, 340).

8. Electronic smoking device of claim 5 wherein the bottleneck (141) is formed by two through holes through the plate (140, 340).

9. Electronic smoking device of one of the preceding claims wherein the mouthpiece portion (14) is conductive and wherein the atomizer (126) is electrically connected between the plate (240, 340) and the mouthpiece portion (14).

10. Electronic smoking device of claim 9 further comprising, arranged within the main body (12) an electric energy providing element (18) electrically connected to the main body (12) and a further conductive part (50) electrically connected to the electric energy providing element (18).

11. Electronic smoking device of claim 10 wherein the mouthpiece portion (14) and the main body (12) are configured for being electrically connected to each other and wherein, when the mouthpiece portion (14) and the main body (12) are fitted together, the conductive part (240, 340) is in electrical contact with the further conductive part (50).

12. Electronic smoking device of claim 10 or 11 wherein an isolation element (60) is arranged between the mouthpiece portion (14) and the conductive part (240, 340) and wherein a further isolation element (70) is arranged between the main body (12) and the further conductive part (50).

13. Electronic smoking device of one of claims 10-12 wherein at least one of the main body (12) and the further conductive part (50) is electrically connected to the electric energy providing element (18) via the control electronics (22) configured to establish an electrical connection between the electric energy providing element (18) and the at least one of the main body (12) and the further conductive part (50) in response to one of a push button being pushed and the airflow sensor (24) sensing an airflow.

14. Electronic smoking device of one of the preceding claims wherein the airflow sensor (24) is a pressure sensitive switch which is activated by a pressure difference between two ends of the airflow sensor (24).

15. Electronic smoking device of one of the preceding claims wherein the mouthpiece portion (14) comprises an external screw thread (11) and the main body (12) comprises a corresponding internal screw thread (21).
